(19) **Europäisches Patentamt**

**European Patent Office**

**Office européen des brevets**

(11) **EP 1 367 135 A1**

(12) **EUROPEAN PATENT APPLICATION**

(43) Date of publication:
03.12.2003 Bulletin 2003/49

(51) Int Cl.⁷: $C12Q\ 1/37$

(21) Application number: **02011945.9**

(22) Date of filing: **29.05.2002**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI**<br><br>(71) Applicant: **Pentapharm AG**<br>**4052 Basel (CH)** | (72) Inventor: **Calatzis, Andreas**<br>**81673 Muenchen (DE)**<br><br>(74) Representative: **Braun, André et al**<br>**BRAUN & PARTNER**<br>**Patent-, Marken-, Rechtsanwälte**<br>**Reussstrasse 22**<br>**4054 Basel (CH)** |

(54) **Improved method for the assessment of thrombin formation in blood or plasma**

(57) The present invention is directed to a kit and a method for a fast and direct determination of the initial thrombin activity in a blood or plasma sample. The kit comprises at least one activator of the plasmatic coagulation system and a thrombin substrate with a $K_M$ of 1 - 200 μM in a relatively low concentration.

EP 1 367 135 A1

**Description**

The present invention relates to an improved method for determining the onset of thrombin formation and the initial dynamics of thrombin formation which has been present in a sample of plasma or blood and also a kit for use in said method.

Background of the Invention

It is essential for survival that a wound stops bleeding and that childbirth be compatible with survival of the mother, i.e. that the body possesses an adequate mechanism for hemostasis. If however arrest of blood flow occurs in intact vessels, normal circulation is impaired, which is deleterious to normal function. Tissues and organs can die when blood supply is arrested for too long. Nature has provided an admiringly efficient and extremely complicated mechanism, the hemostatic system, to ensure the seemingly contradictory functions: adequate blood flow in normal vessels and prompt arrest of bleeding in damaged ones. It is not surprising that the hemostatic mechanism is a very complicated and extremely fine tuned one, replete with checks and balances (Hemker H C, Beguin S. Phenotyping the clotting system. Thromb Haemost 2000; 84: 747-51).

Hemostasis is brought about by the interplay between the minute blood cells, the blood platelets, and a set of proteins of the blood plasma, the coagulation system. Having past a damaged part of the vessel wall, platelets stick to the bare tissue in the wound and create a scenery in which blood can clot without the clot being washed away by the flowing blood. The interactions between the wound and the blood lead to the formation of thrombin. Thrombin is responsible for a whole concert of reactions, of which the actual clotting, i.e. solidification of the blood is only one: Thrombin activates the platelets and acts on the cell of the vessel wall. Activated platelets, in their turn, foster thrombin formation. Thrombin partakes in a whole set of positive and negative feedback reactions. (Hemker et al US 5,192,689)

Essential to the understanding is the concept of proenzyme-into-enzyme conversion. An enzyme is a protein capable to enhance a very specific chemical reaction. Proteolytic enzymes are proteins that can cut other proteins in pieces. They can therefore be extremely dangerous and usually are formed and transported in the body as proenzymes. Proenzymes are usually larger than enzymes and cannot, as such, do any harm.

Only when they are cut at a specific place does their enzyme character appear. E.g. enzymes that are to digest the proteins of our food are formed as proenzymes in the pancreas and are only activated when secreted in the gut. The clotting mechanism is based on an ordered series of proenzyme - enzyme conversions. The first one of the series is transformed into an active enzyme that activates the second one, which activates the third one. In this way a few molecules in the beginning of the series create an explosion of the final active enzyme: thrombin. This mechanism is often referred to as the coagulation cascade.

Thrombin, in the blood, does not live long; otherwise the slightest wound could make all the blood clot. Its survival time in plasma is only a few minutes, due to the fact that it is bound by antithrombins, suicidal plasma proteins that inactivate thrombin by binding to it irreversibly. Antithrombins also act as regulatory proteins which can inactivate the first traces of thrombin before they can augment thrombin generation by the positive feedback reactions.

The cascade sequence only explains the explosive formation of thrombin, however, clotting is not as simple as that, the output is finely tuned by a web of positive and negative feedback reactions. Coagulation starts when a vessel is wounded. A vessel is wrapped in a layer that is full of cells containing Tissue Factor (TF) (Mann KG: Biochemistry and physiology of blood coagulation. Thromb Haemost 1999 Aug;82(2):165-74). After injury this TF is exposed to blood, it binds with factor VIIa to form the first enzyme: TF:VIIa. This activates factor X to Xa, which in turn forms thrombin. All the plasma proteins involved in coagulation are clotting factors, indicated by Roman numerals. When a proenzyme is activated into an enzyme, an "a" is added to the number. Factor Xa is not a very efficient enzyme; it needs the help of activated factor V (a helper protein or cofactor) that helps to speed up prothrombin activation a thousand fold. Both factors bind to a phospholipid surface that also binds prothrombin and thus favours the meeting of substrate and enzyme. (Xi M, Beguin S, Hemker H C. The relative importance of the factors II, VII, IX and X for the prothrombinase activity in plasma of orally anticoagulated patients. Thromb Haemost 1989; 62: 788-91)

This offers the possibility to regulate the function of factor Xa, and hence thrombin production in space and time. Factor V is activated by thrombin into factor Va. Xa and Va bind onto a phospholipid surface and form the prothrombinase complex which very efficiently converts more prothrombin into thrombin. Thrombin also binds to thrombomodulin. This complex converts protein C (again a proenzyme) into its activated (enzyme) form: APC. APC in turn attacks factor Va and inactivates it (by converting it to factor Vi). (Rosing J, Hemker HC, Tans G. Molecular biology and pathophysiology of APC resistance: current insights and clinical implications. Semin Thromb Hemost. 1998;24(4): 329-35.) VIIa:TF not only activates factor X but also factor IX (nine). Thrombin also activates factor VIII that, just like factor V, is a helper protein. Factor VIIIa and IXa form a complex called tenase, that produces more X. Like factor Va, factor VIIIa is degraded by activated protein C. Like the Xa-Va complex, the IXa-VIIIa complex only functions well when adsorbed onto a phospholipid surface.

Blood contains platelets (thrombocytes). As soon as they find an injury they bind to the exposed tissue (collagen) and, by this binding and the contact with thrombin, they activate. This results in a change of the surface of the platelets, that now becomes able to bind clotting factors such as the couples Xa-Va and IXa-VIIIa. This binding is essential for their proper functioning. The platelet surface serves as a two-dimensional space where the clotting factors can find each other and more easily perform their reactions. This adsorption again increases thrombin formation by about a thousand fold. So without such a surface clotting will not occur. This provides the Localization Dimension: clotting only occurs where it is needed and remains contained to that area (Beguin S, Keularts I. On the coagulation of platelet-rich plasma. Physiological mechanism and pharmacological consequences. Haemostasis 1999; 29: 50-7).

The result of this all is that a complicated web of positive and negative feedback loops governs thrombin production. These mechanisms are so complicated as to make it (even theoretically) impossible to describe them in a mathematical model. Therefore it becomes extremely difficult to predict with any accuracy how the system will quantitatively answer to changes to one or more of its reactants.

Drugs that are designed to inhibit thrombosis by selectively attacking one single enzyme may in practice work out completely different than expected. The consequence is that the coagulation potential of the blood has to be measured rather than to try to predict it.

The conventional way of assessing the hemostatic system is to activate hemostasis (using different activators) and to assess the time it takes for blood or plasma until clotting is detected ("clotting time"). Alternatively the concentration of certain components is determined or the activity of certain components is assessed in artificial isolated systems (single factor activities). By all these approaches the dynamics of thrombin activation are not directly assessed. Clotting times only represent the lag phase before thrombin generation starts and therefore only tell a minor part of the story. The extent of the hemostatic-thrombotic reaction is also critically determined by the dynamics of thrombin formation.

In the past different strategies for quantifying the dynamics of thrombin formation have been developed. Most known are the techniques developed by Hemker and Beguin (US Patent No. 5,192,689) et al and modifications of these methods by other authors.

The method of Hemker relies on the determination of the so-called "endogenous thrombin potential" (ETP) in a sample of either clotting blood or plasma. A thrombin formation activator is added to the sample together with a thrombin substrate, wherein the amount and also the kinetic properties of said thrombin substrate are chosen such that the amount of thrombin generated in the sample cannot completely consume said thrombin substrate. Thrombin formed during the clotting reaction consumes said substrate, thereby to produce a conversion product. The amount of said conversion product is determined, and from this the endogenous thrombin potential in the sample is calculated (Hemker H C, Wielders S, Kessels H, Beguin S. Continuous registration of thrombin generation in plasma, its use for the determination of the thrombin potential. Thromb Haemost 1993; 70: 617-24).

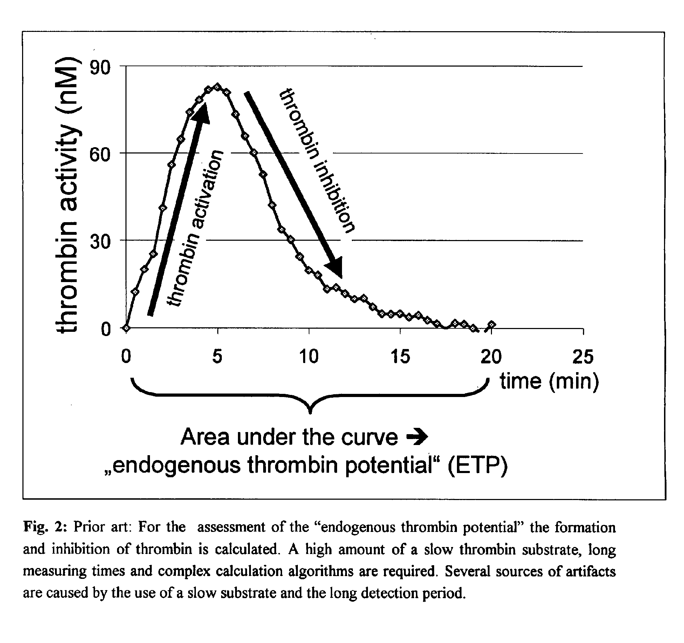

Typically the ETP is calculated based on the area under the curve of the thrombin activity (Fig. 2). The thrombin activity is calculated based on the first derivative of the production of the conversion product of the thrombin substrate.

The ETP method by Hemker relies on the continuous assessment of thrombin formed *in vitro,* - from its formation until it is inhibited by the endogenous inhibitory substances in the blood or by added inhibitors in the reagent (Fig. 2). The ETP method is based on the concept that the hemostatic activity that develops in a wound or thrombus is essentially dependent upon the number of "man-hours" of thrombin that can develop in blood. According to this concept the amount of thrombin that generates as well as the length of time that it is active both count. The amount of work that can potentially be done by thrombin is reflected in the area under the curve that describes the concentration of thrombin in time during clotting, that is, the ETP. Another important property is the time it takes until thrombin formations starts, i.e. the lag time. This also happens to be the clotting time, because the clot appears when roughly 1% of thrombin is formed. One misses out on a lot of information if he just looks at the clotting time as a determinant of coagulability: after the clot formation most of the thrombin action is still to come (Hemker H C, Beguin S. Thrombin generation in plasma: its assessment via the endogenous thrombin potential. Thromb Haemost 1995; 74: 134-8).

**The following strategies have been developed for assessing the ETP:**

Use of chromogenic thrombin substrates

Chromogenic substrates are peptides which are coupled to chromophoric groups. They are usually applied in aqueous solution. When these chromophoric groups are split from the peptide by enzymatic action the optical density of the solution rises and this can be detected photometrically. Chromogenic substrates are used in many diagnostic methods for the assessment of the activity of certain enzymes. The commonly used chromogenic substrates for thrombin are readily split by thrombin action and are thus rapidly consumed by its action. For the use in the ETP method chromogenic substrates have been applied which are slowly converted by thrombin. These are either substrates which were originally developed for other enzymes (e.g. the substrate S-2222, which was developed for the assessment of FXa activity) or substrates developed especially for the assessment of the ETP (Rijkers D T, Hemker H C, Tesser G I. Synthesis of peptide p-nitroanilides mimicking fibrinogen- and hirudin- binding to thrombin. Design of slow reacting thrombin substrates. Int J Pept Protein Res 1996; 48: 182-93.).

The chromophors (e.g. p-nitro-anilin [p-NA]) used in the chromogenic substrates are typically assessed using light with a wavelength of 405 nm. Both fibrin and platelets in aqueous solutions interfere with the assessment of the release of the chromophors of chromogenic substrates at 405 nm. When thrombin is formed it splits two pieces of the fibrinogen molecule (so called fibrino-peptides A and B). The resulting molecule is called fibrin. It spontaneously polymerizes to form fibrin polymers (fibrin strands), which are the covalently cross-linked by factor XIIIa (a clotting factor which is also activated by thrombin). This mechanism (fibrin formation and polymerization) is an integral part of the hemostatic process in the body.

In order to assess the ETP using chromogenic substrates the sample must either be depleted from fibrinogen (defibrination) before the analysis or substances which interfere with fibrin polymerization must be added (Wielders S, Mukherjee M, Michiels J, Rijkers D T, Cambus J P, Knebel R W, Kakkar V, Hemker H C, Beguin S. The routine determination of the endogenous thrombin potential, first results in different forms of hyper- and hypocoagulability. Thromb Haemost 1997; 77: 629-36). The defibrination of the sample can be performed using snake venoms which split fibrinogen. The resulting fibrin clot can be removed for the plasma by centrifugation or can be removed manually. However this requires a separate step for the preparation of the sample, which can not be performed by the standard laboratory analyzers. As a separate step which has to be performed by the laboratory staff instead of the automated equipment it significantly enhances the cost of such an analysis. The defibrinated sample cannot be used for the standard analysis of the coagulation system, but can only be used for the ETP method. In addition it is also unclear whether the removal of the artificially produced fibrin clot also extracts any relevant substances from the plasma sample that might be relevant for the function of the coagulation cascade (Kumar R, Beguin S, Hemker H C). The influence of fibrinogen and fibrin on thrombin generation is an evidence for feedback activation of the clotting system by clot bound thrombin. Thromb Haemost 1994; 72: 713-21).

Substances are known that inhibit fibrin polymerization. The most commonly used substance is the peptide H-Gly-Pro-Arg-Pro-OH AcOH. The use of this peptide in the ETP method has been introduced by Stuerzebecher et al and has the major advantage that the sample has not to be defibrinated before the analysis (Prasa D, Svendsen L, Sturzebecher J. Inhibition of thrombin generation in plasma by inhibitors of factor Xa. Thromb Haemost 1997 Oct;78 (4): 1215-20; Prasa D, Svendsen L, Sturzebecher J. The ability of thrombin inhibitors to reduce the thrombin activity generated in plasma on extrinsic and intrinsic activation. Thromb Haemost 1997 Mar;77(3):498-503).

However quite high concentrations of H-Gly-Pro-Arg-Pro-OH AcOH must be applied and the inhibition of fibrin polymerization is often incomplete, especially in samples with high fibrinogen concentrations. As fibrinogen is a so-called acute phase protein it can reach very high concentrations in the plasma of patients (up to 10 g/l). Thus many patients whose ETP might be relevant have elevated fibrinogen concentrations which can disturb the assessment of the ETP using fibrin polymerization inhibitors.

Use of fluorogenic substrates

Another strategy for assessing the ETP - which is today more popular than the chromogenic method - is the use of fluorogenic substrates (Hemker H C, Giesen P L, Ramjee M, Wagenvoord R, Beguin S. The thrombogram: monitoring thrombin generation in platelet-rich plasma. Thromb Haemost 2000; 83:589-91). Fluorogenic substrates are analogous to chromogenic substrates. The difference is that the substrates release upon enzymatic action a group which can be determined using a fluorometer. Fluorometers have a high sensitivity for detecting low amounts of substances with fluorescenic properties. Fluometric assessment of the ETP also has the advantage that fibrin or platelets do not interfere with the analysis. I.e. no measures have to be performed for defibrinating the sample or for inhibiting fibrin polymerization. In addition platelet-rich plasma can be used for the analysis, which allows the assessment of the interaction of the plasmatic factors (enzymes) and platelets during thrombin formation (Keularts I M, Hamulyak K, Hemker H C, Beguin S. The effect of DDAVP infusion on thrombin generation in platelet-rich plasma of von Willebrand type 1 and in mild haemophilia A patients. Thromb Haemost 2000; 84: 638-42).

However the use of fluorogenic substrates has also significant disadvantages: The standard laboratory equipment used for analysis of the coagulation system does to support fluorometric analysis. Thus the analysis requires additional expensive instrumentation (fluorometer). This produces significant costs and is a major obstacle for the introduction of this method. In addition the trend of the last decade has been to implement all coagulation tests wherever possible on one analyzer in order to simplify the testing procedure and minimize labour costs. The use of a separate instrument for the ETP method reduces significantly its applicability as a routine method.

The fluorometers currently used for the ETP method do not provide an automated pipetting of the ETP procedure, thus for performing the procedure several manual pipetting steps are required, which again enhances the cost of the procedure and complicates the introduction of the method in routine laboratories.

General aspects of the ETP method by Hemker As mentioned the ETP method by Hemker and Beguin (US-Patent No. 5,192,689) requires the use of a slow substrate for thrombin, i.e. a substrate that has a high $K_M$ and is thus slowly converted by thrombin formed during by the coagulation cascade. Hemker explains that it is necessary to use a thrombin substrate with a high $K_M$ in order to minimize artificial effects of the substrate on the coagulation cascade. Most coagulation factors are- like thrombin -serine proteases and have homologies to thrombin. According to Hemker in order to assess thrombin formation in a valid way one has to use a substrate with a high $K_M$ which will thus not lead to a major interference with the coagulation cascade.

However the use of a slow thrombin substrate leads to a relatively weak signal. This complicates the precise assessment of the "lag phase" i.e. the time from the start of the analysis until the detection of free thrombin in the sample-reagent mixture.

The use of a slow substrate together with the requirement to assess thrombin activation and inactivation lead to very long measuring times (typically 30 minutes) and to the need of high substrate concentrations, which both enhance the costs of the analysis. For an analysis in a routine setting measuring times of 30 minutes have two negative effects: The turn-around time is significantly enhanced, thus limiting the usefulness of the assay for immediate therapeutic decisions. The analyzer is blocked for a significant time period.

The ETP method by Hemker is based on the determination of the area under the curve (AUC) of thrombin activity, i.e. on the "man hours" that are performed by the thrombin molecules from their activation to their inactivation. Several inhibitory substances inactivate thrombin *in vitro.* One is antithrombin, an inhibitor which forms irreversible complexes with thrombin and other serine proteases and whose action is enhanced by heparins. Another is heparin co-factor II, a more specific inhibitor, whose anti-thrombin action is also enhanced by heparins. Thrombin is also inhibited by less specific inhibitors in plasma. One of the most important is alpha-2-macroglobulin (alpha2-m). Alpha-2-m does not bind to the active center of thrombin (as most other inhibitors do), but incorporates thrombin like a cage. Thrombin which is bound to alpha2-m loses its biological activities: it does not activate platelets and does not split fibrinogen, factor V, factor VIII, factor XI or factor XIII. However it still can split the (small) chromogenic or fluorogenic substrates and thus mimic the activity of active thrombin. This disturbs the correct assessment of the ETP according to Hemker (Rijkers DT, Wielders SJ, Beguin S, Hemker HC: Prevention of the influence of fibrin and alpha2-macroglobulin in the continuous measurement of the thrombin potential: implications for an endpoint determination of the optical density; Thromb. Res. 1998 Feb 15;89(4):161-9). As the concentration of alpha2-m in the sample varies from individual to individual and is also affected by disease states, complex algorithms are required in order to subtract the activity of alpha2-m-bound thrombin from the total thrombin activity determined (Kessels H, Willems G, Hemker HC. Analysis of thrombin generation in plasma. Comput Biol Med 1994 Jul;24(4):277-88 ).

Methods for the assessment of the thrombin formation in fluids using separate aliquots ("sub-sampling")

In many scientific studies methods are applied that assess the thrombin generation of haemostatic processes using a process called "sub-sampling". Examples are given in:

- Beguin S, Lindhout T, Hemker HC. The effect of trace amounts of tissue factor on thrombin generation in platelet rich plasma, its inhibition by heparin. Thromb Haemost 1989 Feb 28;61(1):25-9

- Kessels H, Beguin S, Andree H, Hemker HC. Measurement of thrombin generation in whole blood--the effect of heparin and aspirin. Thromb Haemost 1994 Jul;72(1) :78-83

- Reverter JC, Beguin S, Kessels H, Kumar R, Hemker HC, Coller BS. Inhibition of platelet-mediated, tissue factor-induced thrombin generation by the mouse/human chimeric 7E3 antibody. Potential implications for the effect of c7E3 Fab treatment on acute thrombosis and "clinical restenosis". J Clin Invest 1996 Aug 1;98(3):863-74

- Butenas S, van't Veer C, Mann KG. "Normal" thrombin generation. Blood 1999 Oct 1;94(7):2169-78

- van 't Veer C, Golden NJ, Kalafatis M, Simioni P, Bertina RM, Mann KG. An *in vitro* analysis of the combination of haemophilia A and factor V (LEIDEN). Blood 1997 Oct 15;90(8):3067-72

In these and many other studies a blood or plasma sample is reacted with an activator of hemostasis. During the activation of coagulation small aliquots are repeatedly extracted from the sample and placed in a buffer solution which contains a chromogenic thrombin substrate (this process is called "sub-sampling"). In each aliquot the thrombin activity is assessed separately. This procedure produces significant effort, as up to 80 samples are analyzed for each experiment and is therefore not applicable for routine use.

Thrombin substrates are also used in various other laboratory methods, e.g. synthetic substrates for thrombin are used in applications in the analysis of hemostasis (Fareed J, Messmore HL, Walenga JM, Bermes EW Jr. Synthetic peptide substrates in hemostatic testing. Crit Rev Clin Lab Sci. 1983;19(2):71-134). Among these applications are methods that assess the antithrombin activity (Blomback M, Blomback B, Olsson P, Svendsen L: The assay of anti-thrombin using a synthetic chromogenic substrate for thrombin. Thromb Res 1974 Nov;5(5):621-32), the heparin concentration (Choo IH, Didisheim P, Doerge ML, Johnson ML, Bach ML, Melchert LM, Johnson WJ, Taylor WF. Evaluation of a heparin assay method using a fluorogenic synthetic peptide substrate for thrombin. Thromb Res Suppl. 1982 Jan 1-15;25(1-2):115-23.), the thrombin or prothrombin activity (Hitomi Y, Kanda T, Niinobe M, Fujii S. A sensitive colorimetric assay for thrombin, prothrombin and antithrombin III in human plasma using a new synthetic substrate. Clin Chim Acta 1982 Mar 12;119(3):157-64), and methods that assess the anticoagulant drug hirudin and other direct thrombin inhibitors (Griessbach U, Sturzebecher J, Markwardt F: Assay of hirudin in plasma using a chromogenic thrombin substrate. Thromb Res Suppl. 1985 Jan 15;37(2):347-50.). Using these methods the activity of the thrombin or prothrombin in the sample is specifically assessed or the inhibition of added thrombin by endogenous or exogenous anticoagulants is determined.

Further methods have been described that apply thrombin substrates in order to assess the prothrombin time: (US4784944 (Kolde): Prothrombin time determining reagent and methods of using and preparing the same) (Ohki M, Tanaka S, Uchida K, Yamaguchi T, Kato H. A basic study on a highly sensitive automated method for hypercoagulable state in plasma, fluorogenic prothrombin time method. Rinsho Byori 1993 Oct;41(10):1153-8) (US4289498 (Baughman): One-stage prothrombin assay and compositions useful therein).

## Summary of the prior art

As aforementioned the conventional way of assessing the hemostatic system is for instance to activate hemostasis and to assess the "clotting time". Alternatively the concentration of certain components is determined or the activity of certain components is assessed in artificial isolated systems (single factor activities). By all these approaches the dynamics of thrombin activation are not directly assessed. "Clotting times" only represent the lag phase before thrombin generation starts.

Furthermore, the use of chromogenic substrates for assessing the "endogenous thrombin potential" (ETP) of a defibrinated sample requires a separate step for the preparation of the sample, which can not be performed by the standard laboratory analyzers. Additionally, the defibrinated sample cannot be used for the standard analysis of the coagulation system, but can only be used for the ETP method. In addition it is also unclear whether the removal of the artificially produced fibrin clot also extracts any relevant substances from the plasma sample that might be relevant for the function of the coagulation cascade. The peptide H-Gly-Pro-Arg-Pro-OH AcOH which inhibits fibrin polymerization can be used in the ETP method. However, quite high concentrations of H-Gly-Pro-Arg-Pro-OH AcOH must be applied and the inhibition of fibrin polymerization is often incomplete, especially in samples with high fibrinogen concentrations.

The use of fluorogenic substrates has also significant disadvantages: The standard laboratory equipment used for analysis of the coagulation system does to support fluorometric analysis. Thus the analysis requires additional expensive instrumentation. Furthermore, the performance of an assay with fluorogenic substrates requires several manual pipetting steps which again enhances the cost of the procedure and complicates the introduction of the method in routine laboratories.

The use of a slow thrombin substrate according to the method of Hemker leads to a relatively weak signal. This complicates the precise assessment of the "lag phase".

The use of a slow substrate together with the requirement to assess thrombin activation and inactivation lead to very long measuring times and to the need of high substrate concentrations, which both enhance the costs of the analysis.

Summarizing, all currently available methods for quantifying the thrombin formation during coagulation processes have several disadvantages, which have limited their use up until now to non-routine research applications.

Thus, the great challenge for the present invention is to provide an improved method for directly, fast determining the onset of thrombin formation and the initial dynamics of thrombin formation in a sample. The problems and disadvantages which are associated with the routine use of prior art methods can be overcome by the subject-matter of the present invention.

## Summary of the invention

The present invention relates to a kit for determining the initial thrombin activity in a blood or plasma sample comprising at least one activator of the plasmatic coagulation system and a thrombin substrate with a $K_M$ of 1 - 200 µM in a relatively low concentration, which will typically be exhausted by the thrombin formed during the analysis within 5 - 600 seconds.

The thrombin substrate may be a chromogenic, a fluorogenic, or an amperogenic thrombin substrate or other substrate for detecting the thrombin activity.

In a preferred embodiment the chromogenic substrate is selected from the group consisting of:

H-D-CHG-Ala-Arg-pNa·2AcOH, H-D-Phe-Pip-Arg-pNA·2HCl, Boc-Asp(OBzl)-Pro-Arg-NH-Mec, pyroGlu-Pro-Arg-pNA·HCl and Bz-Phe-Val-Arg-pNA.

In another preferred embodiment of the present invention the thrombin substrate has a $K_M$ of 1-100 μM, preferably a $K_M$ of 1-50 μM. In a further embodiment of the invention the thrombin substrate has a concentration of 1-1000 μM, preferably 1-500 μM, more preferably 1-250 μM.

The kit according to the present invention may further comprise a substance which interferes with the fibrin gelation. In a preferred embodiment the fibrin gelation is a fibrin polymerization inhibitor. The fibrin polymerization inhibitor can be H-Gly-Pro-Arg-Pro-OH AcOH in a concentration of 0,1-10 mg/ml, preferably 5 mg/ml.

Furthermore, the kit may comprise a coagulation inhibitor. Examples for suitable coagulation inhibitors are Protein C activators (e.g. Protac), Tissue Factor Pathway Inhibitor (or equivalent substances), inactivated factor VIIa (or equivalent substances), Activated Protein C (or equivalent substances), platelet inhibitors (e.g. prostacyclin), FVIII inhibitor (or equivalent substances), FIX inhibitor (or equivalent substances), Heparins and / or heparinoids, Direct thrombin inhibitors, Direct Factor Xa inhibitors and Serine protease inhibitors (e.g. aprotinin).

A further embodiment of the kit may comprise a coagulation accelerant. Suitable coagulation accelerants according to the present invention are Phospholipids of natural or synthetic origin or analogous substances and Calcium chloride or other sources of divalent cations. Activators of the coagulation system which may be used according to the present invention are Tissue Factor or analogous substances, activators of factor X (e.g. snake venoms), activators of factor V (e.g. snake venoms), prothrombin activators (e.g. snake venoms), contact phase activators (e.g. kaolin), activated coagulation factors or analogous substances and platelet activators (e.g. collagen). A preferred activator of the coagulation system is recombinant tissue factor which may be used in a concentration of 2-20000 pg/ml. Another preferred activator of the coagulation system, aluminium silicate, may be used in a concentration of 0.5-10 g/l.

The present invention is further directed to a method which enables the determination of the initial thrombin activity in a blood or plasma sample by the following steps:

a) mixing a blood or plasma sample with at least one activator of the coagulation system and a thrombin substrate in a relatively low concentration,

b) determining the release of the conversion product of the thrombin substrate,

c) calculating at least one value indicative of the coagulation potential which is dependent on the velocity of the consumption of the thrombin substrate, wherein the amount and also the kinetic properties of said thrombin substrate are chosen such that the amount of thrombin generated in the sample will typically completely consume said thrombin substrate within 5-600 seconds.

The thrombin substrate can be a chromogenic, a fluorogenic, or an amperogenic thrombin substrate or other substrate for detecting the thrombin activity. In a preferred embodiment the chromogenic substrate is selected from the group consisting of: H-D-CHG-Ala-Arg-pNa·2AcOH, H-D-Phe-Pip-Arg-pNA·2HCl, Boc-Asp(OBzl)-Pro-Arg-NH-Mec, pyroGlu-Pro-Arg-pNA·HCl and Bz-Phe-Val-Arg-pNA.

Furthermore, the conversion product can be detected optically at 405 nm.

Thrombin substrates which have a $K_M$ of 1-200 μM, preferably 1-100μM, more preferably 1-50 μM can be used in accordance with the present invention.

In a preferred embodiment the mixture of at least one activator of the coagulation system and the thrombin substrate further comprises a substance which interferes with the fibrin gelation in step (a), e.g. a fibrin polymerization inhibitor. One example for a fibrin polymerization inhibitor which is useful in the practice of the present invention is H-Gly-Pro-Arg-Pro-OH AcOH.

In another preferred embodiment the mixture of step a) may comprise a coagulation inhibitor. Examples for suitable coagulation inhibitors are Protein C activators (e.g. Protac), Tissue Factor Pathway Inhibitor (or equivalent substances), inactivated factor VIIa (or equivalent substances), Activated Protein C (or equivalent substances), platelet inhibitors (e.g. prostacyclin), FVIII inhibitor (or equivalent substances), FIX inhibitor (or equivalent substances), Heparins and / or heparinoids, Direct thrombin inhibitors, Direct Factor Xa inhibitors and Serine protease inhibitors (e. g. aprotinin).

In a further embodiment the mixture of step a) may comprise a coagulation accelerant. Suitable coagulation accelerants according to the present invention are Phospholipids of natural or synthetic origin or analogous substances and Calcium chloride or other sources of divalent cations. Activators of the coagulation system which may be used according to the present invention are Tissue Factor or analogous substances, activators of factor X (e.g. snake venoms), activators of factor V (e.g. snake venoms), prothrombin activators (e.g. snake venoms), contact phase activators (e.g. kaolin), activated coagulation factors or analogous substances and platelet activators (e.g. collagen).

Furthermore, the volume ratio of sample and reagents may be higher than 1:10.

Definitions

As used herein the following terms will have the meanings indicated:

**Antithrombins** are suicidal plasma proteins which inactivate thrombin by binding to is irreversibly. They act as regulatory proteins which can inactivate the first traces of thrombin before they can augment thrombin generation by the positive feedback reactions. The action of some Antithrombins is enhanced by heparins.

**"Clotting time"** is the time it takes for blood or plasma until clotting is detected after induction of hemostasis.

**Chromogenic substrates** are peptides which are coupled to chromophoric groups. When these chromophoric groups are split from the peptide by enzymatic action the optical density of the solution rises and this can be detected photometrically.

**Fluorogenic substrates** can be uses analogous to chromogenic substrates. The difference is that the substrates release upon enzymatic action a group which can be determined using a fluorometer.

The **"endogenous thrombin potential" (ETP)** of a sample can be determined in clotting blood or plasma. A thrombin formation activator is added to the sample together with a thrombin substrate, wherein the amount and also the kinetic properties of said thrombin substrate are chosen such that the amount of thrombin generated in the sample cannot completely consume said thrombin substrate. Thrombin formed during the clotting reaction consumes said substrate, thereby to produce a conversion product. The amount of said conversion product is determined, and from this the endogenous thrombin potential in the sample is calculated.

**Activator of the coagulation system -** Several substances are known that can activate hemostasis. Activators of the plasmatic coagulation system induce the cleavage of coagulation factors to their active forms. E.g. Tissue factor forms a complex with activated factor VII. This complex activates factor X and factor IX. Other substances (e.g. collagen) activate the blood platelets. Upon activation platelets secrete substances, which activate coagulation and present adhesive receptors on their surface. In addition the composition of the platelet surface changes in order to allow the attachment of clotting factors.

**Thrombin substrates** are substances, which are split by thrombin and releases groups which can be quantitatively detected. Examples are chromogenic thrombin substrate (the conversion product is detected photometrically) or fluorogenic thrombin substrates (the conversion product is detected using fluorometric analysis).

**A coagulation accelerant** is a material or substance or mixture of such which greatly speeds up the rate of thrombin formation. Phospholipids and calcium ions are coagulation accelerants as they promote the formation of complexes of coagulation factors. In these complexes (e.g. the prothrombinase complex) the activation of coagulation factors is significantly accelerated.

**The first derivative** of a curve reflects the gradient of the curve during the reaction. The first derivative of the optical density of the sample-reagent mixture during the analysis of coagulation using a thrombin substrate reflects the thrombin activity during time.

**Detailed description of the invention**

Quite unexpectedly we found that an assay system which uses in combination an activator of the plasmatic coagulation system, a blood or plasma sample and a fast thrombin substrate in a relatively small concentration (kinetics and amount of the thrombin substrate are chosen such as it will be typically consumed within 5-600 seconds after thrombin activation) allows a much more precise, inexpensive and fast determination of the velocity of thrombin activation in the sample than previously applied methods.

The assay system is less dependent on the thrombin inhibition phase, than previous methods for the assessment of thrombin activation, which has several advantages and enhances the physiological relevance of the assay.

When a chromogenic thrombin substrate is applied a fibrin polymerization inhibitor (or an analogous substance) is applied in order to suppress fibrin gelation which would interfere with the conversion of the chromogenic substrate. However, if fluorogenic detection is applied (or other detection methods, which are not disturbed by the fibrin gelation) then this component is not required for the inventive assay.

For performing the assay according to the present invention the following reagents are required:

- an activator of the plasmatic coagulation system (e.g. recombinant tissue factor in a concentration of 200 ng/ml sample)

- a chromogenic substrate with a $K_M$ of 1-200 μM (e.g. H-D-CHG-Ala-Arg-pNa·2AcOH at concentration of 250 μM/ml sample)

- optionally coagulation accelerants (e.g. phospholipids at a concentration of 50 μg/ml sample, $CaCl_2$ at a concentration of 25 mM /ml sample)

- optionally coagulation inhibitors (e.g. activated protein C at a concentration of 1 U/ml)

- optionally a fibrin polymerization inhibitor (e.g. H-Gly-Pro-Arg-Pro-OH AcOH in a concentration of 5 mg/ml sample)

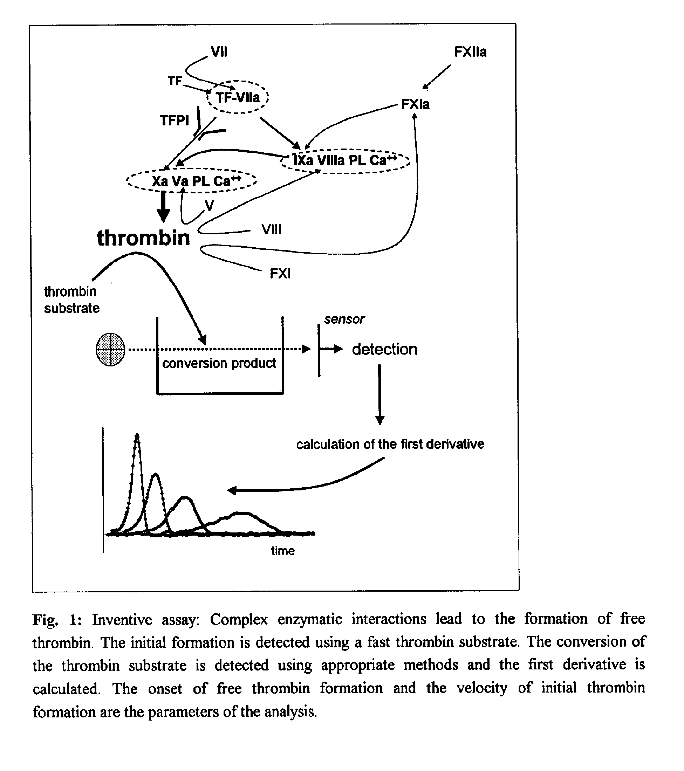

For the analysis of the reaction the conversion of the thrombin substrate is recorded and at least one parameter which depends on the velocity of the conversion of the thrombin substrate is calculated (Fig. 1).

Two or several (or all) of these reagents can be combined in one reagent in order to reduce the number of pipetting steps required. When several components are combined in one reagent it must be evaluated whether the solutions are compatible.

In order to perform the assay procedure a blood or plasma sample is mixed with said reagents and the generation of the conversion product must be determined. If a chromogenic substrate is applied the reaction can be detected optically at 405 nm (in this case only plasma can be analyzed). If a fluorogenic substrate is applied platelet poor or platelet rich plasma can be analyzed. If a amperogenic or equivalent substrate is applied (i.e. when no optical signal is generated) then blood or plasma can be analyzed.

The first derivative of the reaction curve is calculated (e.g. by calculating the increase of optical density over each 4 seconds). The first derivative of the reaction curve reflects the conversion of the thrombin substrate during the assay. The velocity of the conversion of the thrombin substrate depends on whether thrombin is produced fast (so-called "burst" kinetics) or slowly during the reaction. We define the maximum of the first derivative as the "initial thrombin activity" (2af). Using a commercially available calibration plasma the initial thrombin activity (2af) can be converted to a % scale, in which 100% corresponds to a normal blood sample and 0% reflects no thrombin activation at all.

Using the method according to the present invention the thrombin activation can be determined using a fast and inexpensive method, using standard instrumentation. Compared to previous methods the assay is much less time consuming (5-10 minutes, compared to 30 minutes of previous methods), requires a much lower quantity of the thrombin substrate (12.5 nmol substrate, compared to up to 450 nmol required for previously applied methods) and can be fully automated on standard equipment.

Most of the examples for the application of the inventive assay presented use chromogenic substrates for the determination of thrombin activity. However also fluorogenic, amperogenic or other substrates can be used for detecting thrombin activity. Although in the examples the reagents are applied in aqueous solution, modifications of the inventive assay can be applied that apply the reagent in dry form. The blood or plasma sample then dissolves the dry reagents and the reaction is again conducted in the liquid phase. For this application part of the reagents can also be immobilized on solid surfaces (e.g. latex beads).

The method according to the present invention can be adapted to the desired application by the choice of the substrate (chromogenic / fluorogenic / amperogenic), by the concentration of the substrate, by the addition of activators (tissue factor, contact activator) or inhibitors (activated protein C, tissue factor pathway inhibitor). It generates a strong optical signal and can be analyzed using simple algorithms (first derivative). By the use of a fast chromogenic substrate the assay focuses on the initial thrombin activation phase. In contrast to this previously applied methods, especially the ETP assay by Hemker et al, determine the thrombin activation <u>and</u> thrombin inhibition phase. According to Hemker et al the "man hours" of thrombin are the determining factor for hemostasis, bleeding or thrombosis. Therefore in the ETP method the area under the curve of the thrombin activity is calculated. This curve, from the physiological point of view, has two parts: the thrombin formation phase and the thrombin inhibition phase. The velocity of thrombin formation in the first part of the curve provides information on whether thrombin is formed fast ("burst kinetics") or slowly. This information, which is not provided by the standard coagulation assays, provides valuable additional information for the assessment of hemostasis. The thrombin inhibition phase as assessed by the ETP *in vitro* is determined mainly by the antithrombin activity and by other endogenous and exogenous anticoagulants in the sample. However in the body the down-regulation of thrombin formation and its inhibition are strongly influenced by endothelial structures (thrombomodulin) and substances released by or attached on the endothelium (e.g. glycosaminoglycans). Thus the thrombin inhibition phase as assessed *in vitro* is of low clinical relevance and obscures the clinical predictivity of the ETP method. In contrast to this the inventive assay, by focusing on the initial thrombin activation phase, provides more clinically relevant information on the thrombin activation than previous methods.

In US4784944 and a method described by Ohki et al (Ohki M, Tanaka S, Uchida K, Yamaguchi T, Kato H. Rinsho Byori 1993 Oct;41(10):1153-8) a thrombin substrate is applied for the assessment of the onset of thrombin formation following activation of coagulation by tissue factor. These methods were not developed for assessing the amount and velocity of the thrombin formed during the reaction (as in the inventive assay), but only in order to assess the timepoint of free thrombin formation.

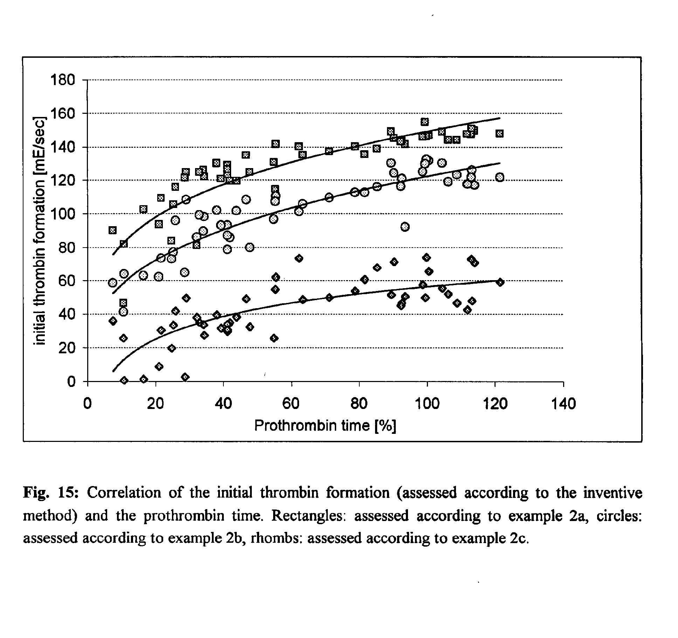

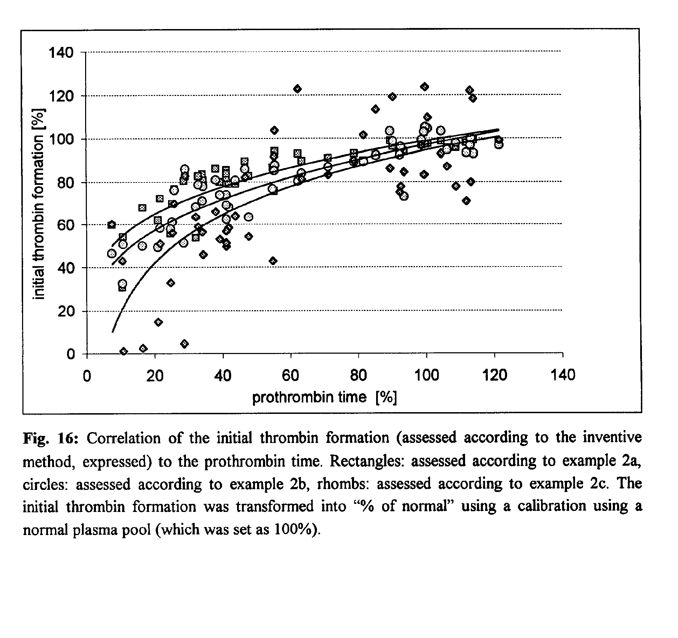

In US4289498 a method for the determination of the prothrombin time is described, which is based on the determination of the exponential rate of the thrombin production in thromboplastin-treated recalcified plasma. For the determination of the rate of thrombin production a thrombin substrate is applied. In this source the thrombin substrate is not used in order to quantify thrombin formation itself, but as a indirect measure for the prothrombin time in a highly diluted assay. Using the diluted assay and the addition of a so-called "serum reagent", which includes various activated coagulation factors, the thrombin formation in the patient sample is modified when compared to the physiological situation. In contrast the inventive assay assesses the thrombin formation using a much less artificial setting. The Baughman source does not teach the use of a relatively small amount of a fast chromogenic substrate for assessing the initial thrombin formation by the hemostatic mechanisms in the sample. In contrast on page 12 line 59-69 Baughman states that the thrombin substrate is preferably applied in stochiometric excess compared to the amount of plasma and that the upper limit of the concentration of the chromogenic substrate is simply the solubility of the substrate in the mixture, which shows that the advantages of the inventive method had not been recognized. In the preferred embodiment in the Baughmann source the chromogenic substrate is used in a concentration of 11.85 mM in relation to the added plasma volume. In the inventive method the chromogenic substrate is preferably used in a concentration of only 250 $\mu$M in relation to the added plasma volume. Fig. 15 and Fig. 16 show that the inventive assay does not simply assess the prothrombin time, as there is considerable variation between the inventive assay and this routine method.

### Description of the figures

**Figure 1** is a schematic representation the inventive assay according to the present invention. It shows the complex enzymatic interactions which lead to the formation of free thrombin. The initial thrombin formation is detected using a fast thrombin substrate. The conversion of the thrombin substrate is detected using appropriate methods and the first derivative is calculated. The onset of free thrombin formation and the velocity of initial thrombin formation are the parameters of the analysis.

**Figure 2** demonstrates the assessment of the "endogenous thrombin potential" (ETP). ETP is calculated based on the area under the curve of the thrombin activity. The thrombin activity is calculated based on the first derivative of the release of the conversion product of the thrombin substrate.

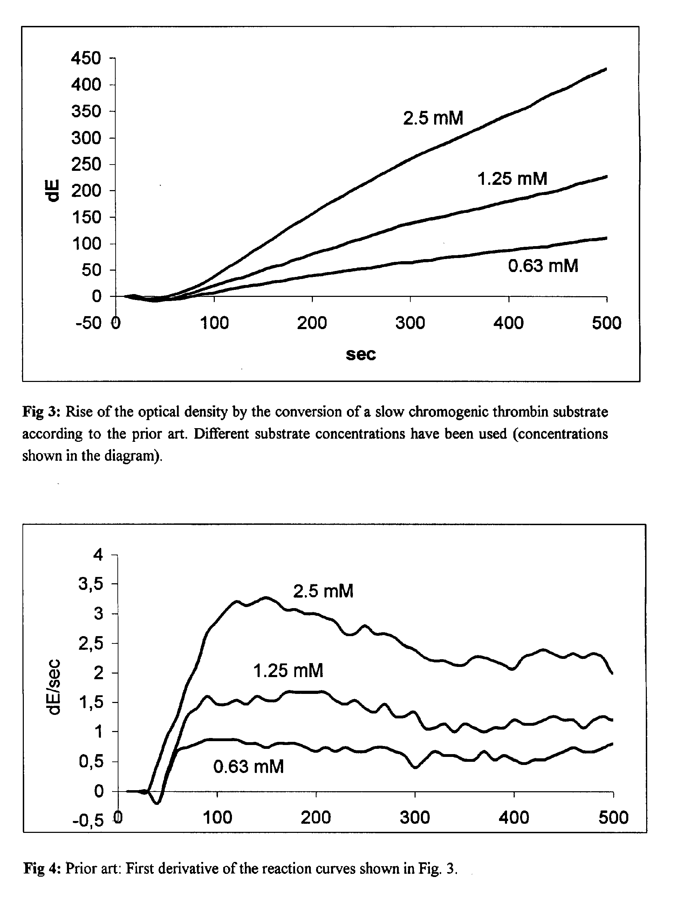

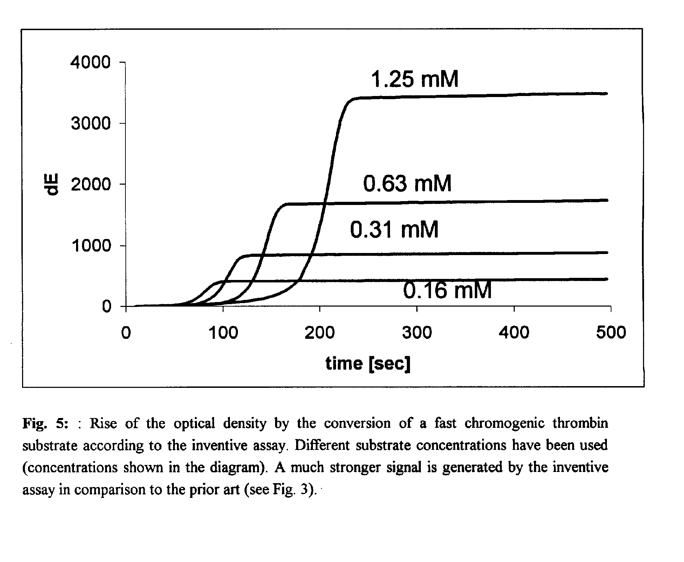

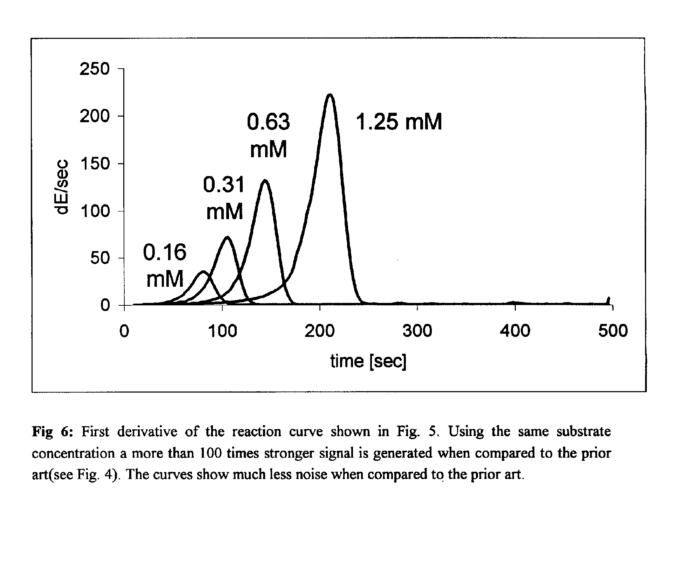

**Figure 3** shows the development of the optical density using previously described methods. Thrombin formation is detected using the slow chromogenic substrate H-$\beta$-Ala-Gly-Arg-pNA$\circ$2AcOH ($K_M$ = 2000 $\mu$M). Fibrin polymerisation was inhibited using the synthetic peptide H-Gly-Pro-Arg-Pro-OH AcOH. Thrombin formation starts after approximately 80 sec. Fig. 4 shows the first derivate of the reaction curve. The different curves show the effect of varying concentrations of the chromogenic substrate. The rise of the optical density does not stop during the reaction, as the chromogenic substrate is not consumed during the reaction. The chromogenic substrate and the fibrin polymerization inhibitor are commercially available from Pentapharm LTD, Basle.

**Figure 4** shows the first derivative of the reaction curves shown in figure 3.

**Figure 5** demonstrates the thrombin formation in the same plasma detected with the method according to the present invention. Using the inventive method a much stronger (20 times stronger) optical signal is generated. The rise of the optical density stops when the substrate is consumed, typically 1-2 min after the onset of thrombin formation. Fig. 6 shows the first derivative of the reaction, which was calculated by determining the rise of the optical density over each 4 seconds. Figure 5 and **Figure 6** show the effect of rising concentrations of the chromogenic substrate H-D-CHG-Ala-Arg-pNa·2AcOH ($K_M$ 15,9 $\mu$M, Pentapharm, Basle) on the detection of the reaction. Rising concentrations of the substrate lead to a rising optical signal. However already the lowest concentrations tested are sufficient for generating a stronger signal than using the previously applied methods. According to the inventive assay the optical signal can be enhanced depending on the concentration of the thrombin substrate applied.

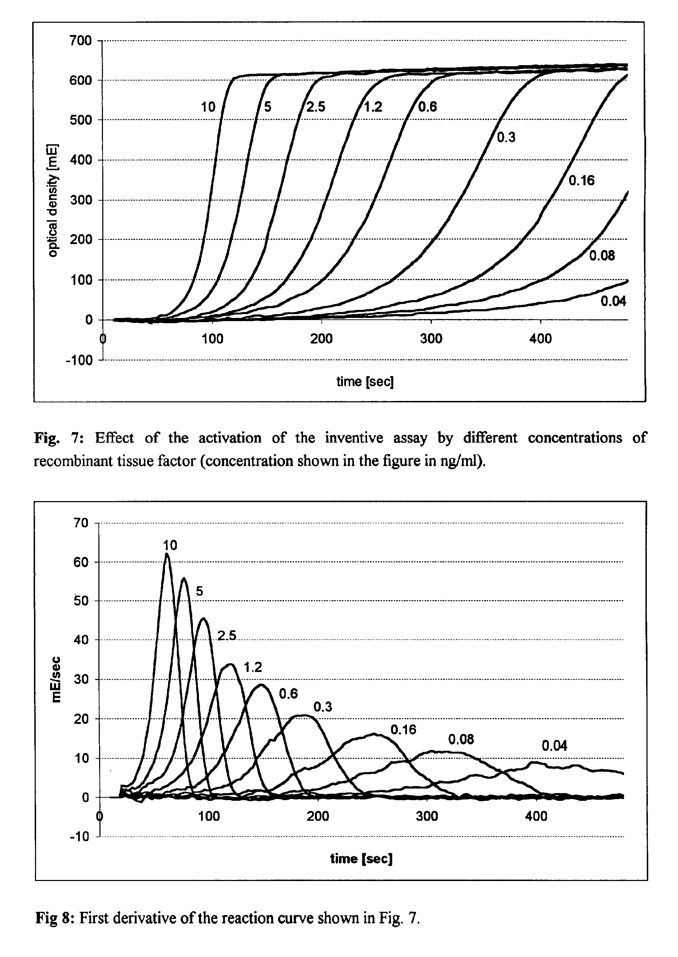

**Figure 7 and 8** show the effect of rising concentrations of the activator of the plasmatic coagulation system on the assessment of the thrombin formation using the inventive assay. Recombinant tissue factor (Instrumentation Laboratory, Kirchheim, Germany) was serially diluted. The numbers in the diagrams show the concentration of recombinant tissue factor [ng/ml sample]. Thus the assay can be adapted as required depending on the activation procedure applied.

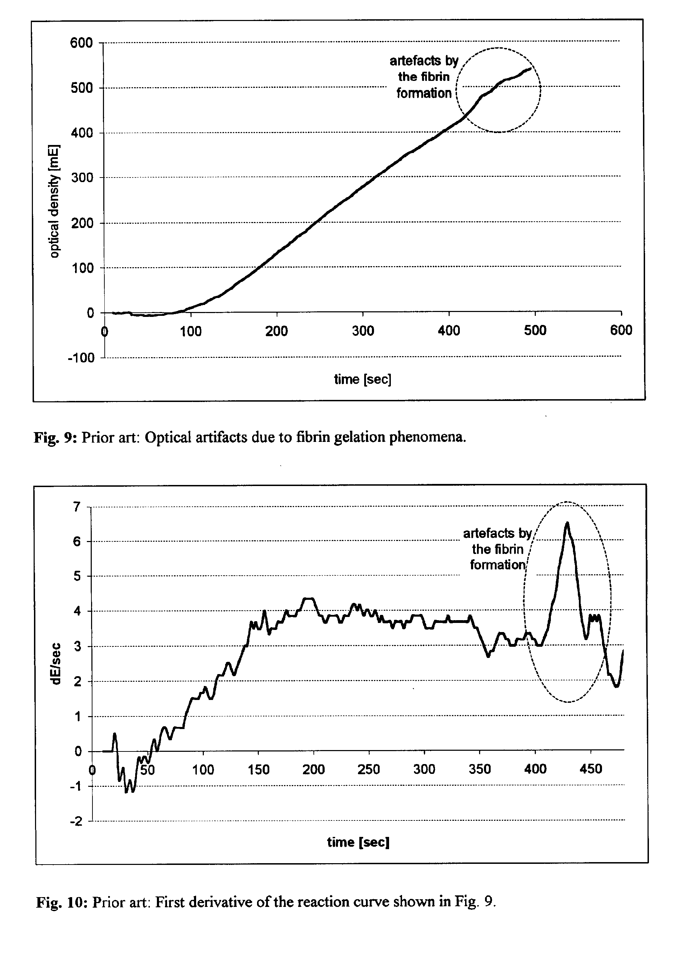

**Figure 9 and 10** demonstrate one of the limitations of the previous art. The thrombin formation was assessed using the slow thrombin substrate H-β-Ala-Gly-Arg-pNA°2AcOH ( 2.5 mM) and the fibrin polymerisation inhibitor H-Gly-Pro-Arg-Pro-OH AcOH (5mg/ml). 400 sec after the onset of thrombin formation optical artefacts are detected, which are even more apparent in the first derivative (Fig. 9). Most likely these artefacts rely on late fibrin gelation phenomena, which occurred in spite of the use of the fibrin polymerisation inhibitor. Fibrinogen is an acute phase protein and is significantly enhanced in many states of disease. Apparently the use of the fibrin polymerisation inhibitor does not completely abolish fibrin gelation in samples with enhanced fibrinogen concentration.

In the inventive method the optical signal is much stronger, so optical artefacts disturb the reaction in a much lower extend. In addition using the inventive method the detection of the thrombin formation ends typically within 1-2 minutes (when the thrombin substrate is consumed), so late fibrin gelation phenomena (as seen in Fig. 9-10) do not affect the measurement.

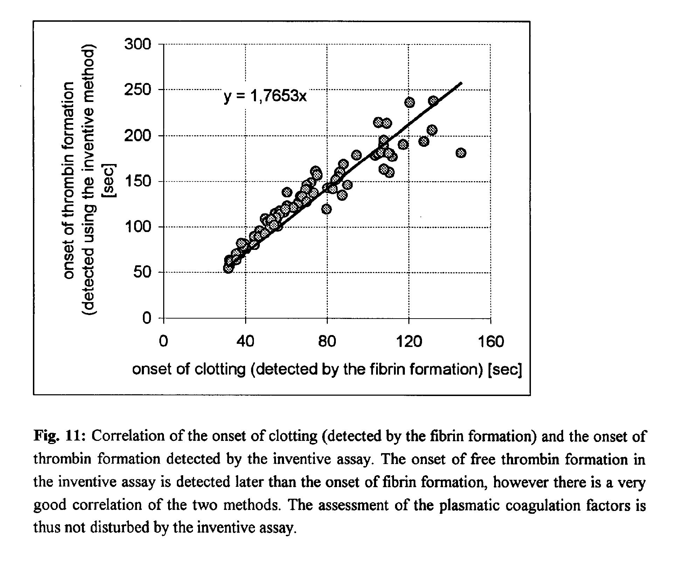

**Figure 11** compares the detection of the onset of coagulation by the fibrin formation and by the inventive method. For this experiment 80 plasma samples from healthy volunteers and hemophilia A patients were clotted by the addition of a contact phase activator (aluminium silicate, Sigma, St. Louis, USA), phospholipids (rabbit brain cephalin, Pentapharm, Basle) and $CaCl_2$ (Sigma, St. Louis, USA) . This procedure is called the "activated partial thromboplastin time" (aPTT). The time from the start of the procedure till clotting is detected is the aPTT. The inventive assay was performed using the identical activation procedure as the aPTT. The difference between the two procedures was the addition of the fast chromogenic substrate (H-D-CHG-Ala-Arg-pNa·2AcOH, 250 μmol/1, Pentapharm) and the fibrin polymerisation inhibitor H-Gly-Pro-Arg-Pro-OH AcOH (5mg/ml, Pentapharm) in the inventive assay. Fig. 10 shows that the addition of the chromogenic substrate delayed the onset of the thrombin formation (as this was proposed by Hemker). However unexpectedly (and in contrast to the publications by Hemker et al) the inhibition of coagulation by the addition of the fast chromogenic substrate resulted in a highly systematic effect, which does not interfere with the correct assessment of hemostasis. The correlation of the two methods was excellent (correlation coefficient 0.95).

**Figure 12** demonstrates the effect of decreasing activities of the coagulation factor FVIII on the assay according to the present invention. The calculation of the onset time and the initial thrombin formation is shown.

**Figure 13** shows the correlation of the factor VIII activity and the initial thrombin formation in hemophilia A patients and healthy volunteers. The initial thrombin formation was transformed into "% of normal" using a calibration using a normal plasma pool (which was set as 100%).

**Figure 14** demonstrates the effect of the addition of FVIII to hemophilia A patients on the initial thrombin formation. The initial thrombin formation was transformed into "% of normal" using a calibration using a normal plasma pool (which was set as 100%).

**Figure 15 and 16** demonstrate different experiments which show the correlation of the initial thrombin formation and the prothrombin time.

### Description of preferred embodiments

#### Example 1

For the assessment of the inventive assay an aqueous solution was prepared containing $CaCl_2$ (25 mM), a chromogenic substrate (250 μM H-D-CHG-Ala-Arg-pNa·2AcOH, Pentapharm, Basle) and a fibrin polymerisation inhibitor (5 mg H-Gly-Pro-Arg-Pro-OH AcOH / ml, Pentapharm, Basle) (solution 1).

A second solution containing a contact activator (aluminium silicate 5 g /1, Sigma, St. Louis, USA) and phospholipids (derived from rabbit brain cephalin, 50 μg/ml, Pentapharm, Basle) was prepared (solution 2).

Platelet poor plasma was prepared from venous citrated blood by centrifugation (20 min at 1500 g).

The procedure was performed as follows:

50 μl platelet poor plasma + 50 μl solution 2 -> 180 sec incubation -> + 50 μl solution 1 -> detection of the optical density at 405 nm over 400 sec

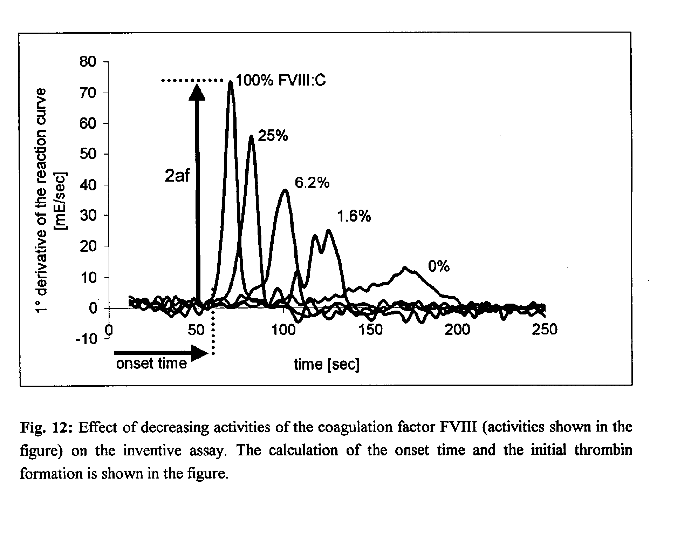

From the reaction curve the first derivative was calculated by determining the rise of optical density over each 4 seconds. The maximum of the first derivative was defined as the initial thrombin activity (2af) (Fig. 12). A commercially available calibration plasma (produced using a plasma pool from healthy volunteers, Instrumentation Laboratory, Kirchheim, Germany) was used for calibrating the results. The initial thrombin activity of the calibration plasma was set as 100% (of normal).

Fig. 12 shows the first derivative of the reaction curve for plasma samples with decreasing factor VIII levels. These plasma samples were prepared by serially diluting the calibration plasma using FVIII deficient plasma (a plasma which has been depleted from factor VIII, commercially available from Dade-Behring, Marburg, Germany). Fig. 11 shows that the thrombin formation as assessed by the inventive assay is highly dependent on the FVIII activity in the sample. FVIII is the coagulation factor, whose concentration or activity is decreased in the hemophilia A patients. This deficiency results in a decreased thrombin formation and therefore in a severe bleeding tendency in these patients.

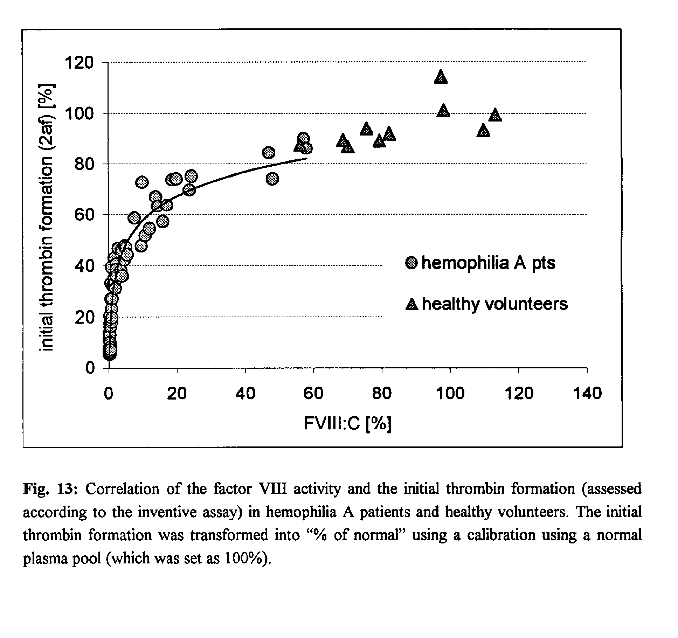

Fig. 13 correlates the results of the inventive assay to the factor VIII activity. Plasma samples from healthy volunteers and hemophilia A patients were analyzed. Results show a very good, non-linear correlation of the two parameters. For plasma samples with a factor VIII activity below 20% (of normal) there is a FVIII-dependent decrease of the thrombin formation as assessed by the inventive assay.

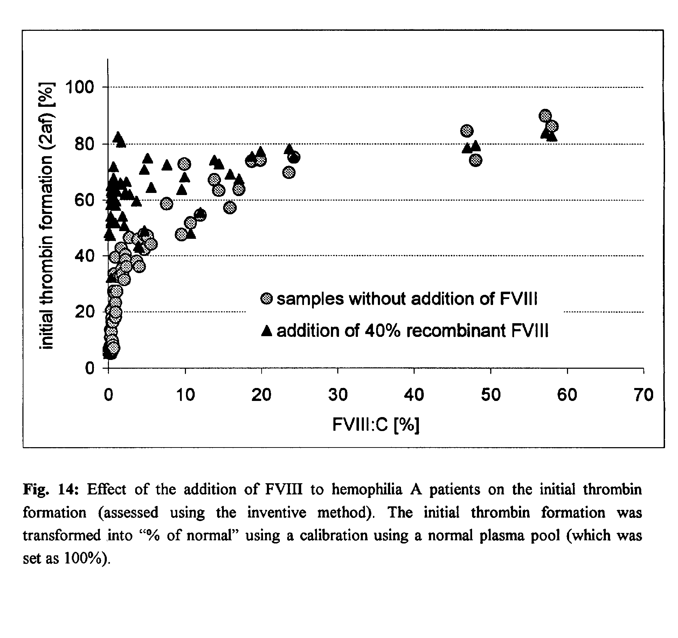

Fig. 14 shows the effect of the addition of recombinant FVIII (Recombinate, Baxter, Vienna, Austria) to the plasma samples on the inventive assay. The addition of FVIII corrected the FVIII deficiency in the samples and resulted in a significant increase of the thrombin formation determined by the inventive assay.

### Examples 2a-c:

For the assessment of the inventive assay an aqueous solution was prepared containing $CaCl_2$ (25 mM), a chromogenic substrate (250 µM H-D-CHG-Ala-Arg-pNa·2AcOH, Pentapharm, Basle), a fibrin polymerisation inhibitor (5 mg H-Gly-Pro-Arg-Pro-OH AcOH / ml, Pentapharm, Basle) and recombinant tissue factor (200 ng/ml, Instrumentation Laboratory, Kirchheim, Germany) (solution 1a).

Solution 1 was prepared by combining $CaCl_2$ (25 mM), a chromogenic substrate (250 µM H-D-CHG-Ala-Arg-pNa·2AcOH), a fibrin polymerisation inhibitor (5 mg H-Gly-Pro-Arg-Pro-OH AcOH / ml) and recombinant tissue factor (20 ng/ml) in aqueous solution.

Solution 1 c (reduced tissue factor content) was prepared by combining $CaCl_2$ (25 mM), a chromogenic substrate (250 µM H-D-CHG-Ala-Arg-pNa·2AcOH), a fibrin polymerisation inhibitor (5 mg H-Gly-Pro-Arg-Pro-OH AcOH / ml) and recombinant tissue factor (2 ng/ml) in aqueous solution.

Solution 2 was prepared A by combining phospholipids (derived from rabbit brain cephalin, 50 µg/ml, Pentapharm, Basle) and HEPES buffer (pH 7.4, 50 mM, Sigma, St. Louis) in aqueous solution.

Platelet poor plasma was prepared from venous citrated blood by centrifugation (20 min at 1500 g).

The procedures were performed as follows:

### Example 2a:

50 µl platelet poor plasma + 50 µl solution 2 -> 180 sec incubation -> + 50 µl solution 1a -> detection of the optical density at 405 nm over 400 sec

### Example 2b:

50 µl platelet poor plasma + 50 µl solution 2 -> 180 sec incubation -> + 50 µl solution 1b -> detection of the optical density at 405 nm over 400 sec

### Example 2c:

50 µl platelet poor plasma + 50 µl solution 2 -> 180 sec incubation -> + 50 µl solution 1c -> detection of the optical density at 405 nm over 400 sec

From the reaction curve the first derivative was calculated by determining the rise of optical density over each 4 seconds. The maximum of the first derivative was defined as the initial thrombin activity (2af). A commercially available calibration plasma (produced using a plasma pool from healthy volunteers, commercially available from Instrumentation Laboratory, Kirchheim, Germany) was used for calibrating the results. The initial thrombin activity of the calibration plasma was set as 100% (of normal).

Citrated plasma from 50 patients with normal or decreased coagulation factor concentrations were assayed. In addition to the inventive method the standard assay prothrombin time was performed (using the reagent Recombiplastin by Instrumentation Laboratory, Kirchheim, Germany). The prothrombin time reflects the coagulation factor concentration of the sample, and is performed by adding to the a sample a reagent containing tissue factor in a very high concentration, phospholipids and $CaCl_2$. The prothrombin time was performed as follows:

50 µl plasma + 100 µl recombiplastin ↦ detection of the clotting time at 405 nm.

Fig. 15 shows the correlation of the initial thrombin activity determined using the inventive assay to the prothrombin time. Using the method according to example 2a a higher thrombin formation was detected when compared to 2b and 2c (the latter method resulted in the weakest thrombin formation). This is due to the decreasing amount of tissue factor which was used in the examples. This also corresponds to the results of the experiment shown in Fig. 7-8.

In Fig. 16 the thrombin activity assessed was transformed into % of normal (by calibrating it against a normal plasma pool) and shown versus the prothrombin time (in % of normal). The initial thrombin activity depends on the coagulation factor activity in the sample (as shown by the significant correlation of the initial thrombin activity versus the prothrombin time) however also considerable variation is seen, especially when the lowest tissue factor activation is applied (example 2c).

Thus information which is not provided by the prothrombin time is determined by the assessment of the initial thrombin formation using the inventive assay.

### Example 3:

For the assessment of the effect of factor VIII supplementation on the initial thrombin formation the following solutions are prepared:

An aqueous solution containing $CaCl_2$ (25 mM), a chromogenic substrate (250 µM H-D-CHG-Ala-Arg-pNa·2AcOH, Pentapharm, Basle) and a fibrin polymerisation inhibitor (5 mg H-Gly-Pro-Arg-Pro-OH AcOH / ml, Pentapharm, Basle) (solution 1).

A second solution containing a contact activator (aluminium silicate 5 g /1, Sigma, St. Louis, USA) and phospholipids (derived from rabbit brain cephalin, 50 µg/ml, Pentapharm, Basle) (solution 2).

A third solution is prepared containing recombinant tissue factor (Baxter, Vienna, Austria) in a concentration of 1 U/ml.

Platelet poor plasma is prepared from venous citrated blood by centrifugation (20 min at 1500 g).

The procedure is performed as follows:

50 µl platelet poor plasma + 50 µl solution 2 + 50 µl solution 3 -> 600 sec incubation -> + 50

µl solution 1 -> detection of the optical density at 405 nm over 400 sec

During the incubation period the added FVIII from solution 3 can be inhibited by anti-FVIII-antibodies in the patient sample. The effect of FVIII supplementation and FVIII inhibition on the thrombin formation is assessed by this method.

### Example 4:

For the assessment of the interaction of plasmatic and cellular components of coagulation the following solutions are prepared:

An aqueous solution containing $CaCl_2$ (25 mM) and a fluorogenic thrombin substrate (H-D-Cha-Ala-Arg-AMC 2 AcOH, 25 µM, Pentapharm, Basle) (solution 1).

AMC: 7-Amino-4-methylcoumarin

A second solution is prepared by combining a contact activator (aluminium silicate 5 g / 1, Sigma, St. Louis, USA) and phospholipids (derived from rabbit brain cephalin, 50 µg/ml, Pentapharm, Basle) in aqueous solution (solution 2).

Platelet rich plasma is prepared from venous citrated blood by centrifugation (20 min at 500 g).

The procedure is performed as follows:

50 µl platelet poor plasma + 50 µl solution 2 -> 180 sec incubation -> + 50 µl solution 1 -> detection of the light emission at about 440 nm (excitation wavelength about 342 nm).

Due to the fluorogenic detection no means for inhibiting fibrin gelation are required. Due to the use of platelet rich plasma in this example the effect of platelet inhibition on thrombin formation can be assessed.

**Example 5:**

For the assessment of the effect of disorders of the protein C system on the initial thrombin formation the following solutions are prepared:

An aqueous solution containing $CaCl_2$ (25 mM), a chromogenic substrate (250 $\mu$M H-D-CHG-Ala-Arg-pNa·2AcOH, Pentapharm, Basle) and a fibrin polymerisation inhibitor (5 mg H-Gly-Pro-Arg-Pro-OH AcOH / ml, Pentapharm, Basle) is prepared (solution 1).

A second solution is prepared by combining a contact activator (aluminium silicate 5 g /1, Sigma, St. Louis, USA) and phospholipids (derived from rabbit brain cephalin, 50 $\mu$g/ml, Pentapharm, Basle) in aqueous solution (solution 2).

A third solution is prepared containing activated protein C (Enzyme Research, South Bend, IN, USA) in a concentration of 1 U/ml (solution 3).

Platelet poor plasma is prepared from venous citrated blood by centrifugation (20 min at 1500 g).
The procedure is performed as follows:

50 $\mu$l platelet poor plasma + 50 $\mu$l solution 2 + 50 $\mu$l solution 3 -> 180 sec incubation -> + 50

$\mu$l solution 1 $\rightarrow$ detection of the optical density at 405 nm over 400 sec

The activated protein C present in solution 3 inactivates FVa from the sample and reduces the thrombin formation. In patients with inherited (e.g. carriers of the factor V Leiden mutation) or acquired disorders of the activated protein C system (e.g. due to oral contraception) the inhibition of thrombin activation is decreased when compared to normal subjects.

**Claims**

1. Kit for determining the initial thrombin activity in a blood or plasma sample comprising:

   at least one activator of the plasmatic coagulation system and a thrombin substrate with a $K_M$ of 1 - 200 $\mu$M in a relatively low concentration, which will typically be consumed by the thrombin formed during the analysis within 5 - 600 seconds.

2. Kit according to claim 1 wherein the thrombin substrate is a chromogenic thrombin substrate.

3. Kit according to claim 2 wherein the chromogenic substrate is selected from the group consisting of:

   H-D-CHG-Ala-Arg-pNa·2AcOH,
   H-D-Phe-Pip-Arg-pNA·2HCl,
   Boc-Asp(OBzl)-Pro-Arg-NH-Mec,
   pyroGlu-Pro-Arg-pNA·HCl and
   Bz-Phe-Val-Arg-pNA.

4. Kit according to claim 1 wherein the thrombin substrate is a fluorogenic thrombin substrate.

5. Kit according to claim 1 wherein the thrombin substrate is a amperogenic thrombin substrate.

**6.** Kit according to any of the preceding claims wherein the thrombin substrate has a $K_M$ of 1-100 µM.

**7.** Kit according to any of the preceding claims wherein the thrombin substrate has a $K_M$ of 1-50 µM.

**8.** Kit according to any of the preceding claims wherein the thrombin substrate has a concentration of 1-1000 µM.

**9.** Kit according to any of the preceding claims wherein the thrombin substrate has a concentration of 1-500 µM.

**10.** Kit according to any of the preceding claims wherein the thrombin substrate has a concentration of 1-250 µM.

**11.** Kit according to any of the preceding claims further comprising a substance interfering with the fibrin gelation.

**12.** Kit according to claim 11 in which the substance which interferes with the fibrin gelation is a fibrin polymerization inhibitor.

**13.** Kit according to claim 11-12 wherein the fibrin polymerization inhibitor is H-Gly-Pro-Arg-Pro-OH AcOH (preferably in a concentration of 0,1-10 mg /ml, more preferably 5 mg /ml).

**14.** Kit according to any of the preceding claims further comprising a coagulation inhibitor.

**15.** Kit according to claim 14 wherein the coagulation inhibitor is selected from the group consisting of:

> a Protein C activator (e.g. Protac),
> Tissue Factor Pathway Inhibitor (or equivalent substances),
> inactivated factor VIIa (or equivalent substances),
> Activated Protein C (or equivalent substances),
> platelet inhibitors (e.g. prostacyclin),
> FVIII inhibitor (or equivalent substances),
> FIX inhibitor (or equivalent substances),
> Heparins and / or heparinoids,
> Direct thrombin inhibitors,
> Direct Factor Xa inhibitors and
> Serine protease inhibitors (e.g. aprotinin).

**16.** Kit according to any of the preceding claims further comprising a coagulation accelerant.

**17.** Kit according to claim 16 wherein the coagulation accelerant is selected from the group consisting of:

> Phospholipids of natural or synthetic origin or analogous substances, Calcium chloride or other sources of divalent cations.

**18.** Kit according to any of the preceding claims, wherein the activators of the coagulation system are selected from the group consisting of:

> Tissue Factor or analogous substances,
> activators of factor X (e.g. snake venoms),
> activators of factor V (e.g. snake venoms),
> prothrombin activators (e.g. snake venoms),
> contact phase activators (e.g. kaolin),
> activated coagulation factors or analogous substances and
> platelet activators (e.g. collagen).

**19.** A method for determining the initial thrombin activity in a blood or plasma sample comprising the following steps:

> a) mixing a blood or plasma sample with at least one activator of the coagulation system and a thrombin substrate in a relatively low concentration,
> b) determining the release of the conversion product of the thrombin substrate,
> c) calculating at least one value indicative of the coagulation potential which is dependent on the velocity of the consumption of the thrombin substrate,

wherein the amount and also the kinetic properties of said thrombin substrate are chosen such that the amount of thrombin generated in the sample will typically completely consume said thrombin substrate within 5-600 seconds.

20. Method according to claim 19, wherein the thrombin substrate is a chromogenic substrate.

21. Method according to claim 20 wherein the chromogenic substrate is selected from the group consisting of:

H-D-CHG-Ala-Arg-pNa·2AcOH, H-D-Phe-Pip-Arg-pNA·2HCl, Boc-Asp(OBzl)-Pro-Arg-NH-Mec, pyroGlu-Pro-Arg-pNA·HCl and Bz-Phe-Val-Arg-pNA.

22. Method according to claim 20 wherein the conversion product is detected optically at 405 nm.

23. Method according to claim 19, wherein the thrombin substrate is a fluorogenic substrate.

24. Method according to claim 19, wherein the thrombin substrate is a amperogenic substrate.

25. Method according to claim 19, wherein the thrombin substrate has a $K_M$ of 1-200 µM.

26. Method according to claim 19, wherein the thrombin substrate has a $K_M$ of 1-100 µM.

27. Method according to claim 19, wherein the thrombin substrate has a $K_M$ of 1-50 µM.

28. Method according to any one of claims 19-27, further comprising a substance interfering with the fibrin gelation in step (a).

29. Method according to claim 28 in which the substance which interferes with the fibrin gelation is a fibrin polymerization inhibitor.

30. Method according to any one of claims 28-29 wherein the fibrin polymerization inhibitor is H-Gly-Pro-Arg-Pro-OH AcOH.

31. Method according to any one of claims 19-30, further comprising at least one coagulation inhibitor in step (a).

32. Method according to claim 31, wherein the coagulation inhibitor is selected from the group consisting of:

        a Protein C activator (e.g. Protac),
        Tissue Factor Pathway Inhibitor (or equivalent substances),
        inactivated factor VIIa (or equivalent substances),
        Activated Protein C (or equivalent substances),
        platelet inhibitors (e.g. prostacyclin),
        FVIII inhibitor (or equivalent substances),
        FIX inhibitor (or equivalent substances),
        Heparins and / or heparinoids,
        Direct thrombin inhibitors,
        Direct Factor Xa inhibitors and
        Serine protease inhibitors (e.g. aprotinin).

33. Method according to any one of claims 19-32, further comprising at least one coagulation accelerant in step (a).

**34.** Method according to claim 33, wherein the coagulation accelerant is selected from the group consisting of:

Phospholipids of natural or synthetic origin or analogous substances,
Calcium chloride or other sources of divalent cations.

**35.** Method according to any one of claims 19-34 wherein the activators of the coagulation system are selected from the group consisting of:

Tissue Factor or analogous substances,
activators of factor X (e.g. snake venoms),
activators of factor V (e.g. snake venoms),
prothrombin activators (e.g. snake venoms),
contact phase activators (e.g. kaolin),
activated coagulation factors or analogous substances and
platelet activators (e.g. collagen).

**36.** Method according to claim 19-35 wherein the volume ratio of sample and reagents is higher than 1:10.

**Fig. 1:** Inventive assay: Complex enzymatic interactions lead to the formation of free thrombin. The initial formation is detected using a fast thrombin substrate. The conversion of the thrombin substrate is detected using appropriate methods and the first derivative is calculated. The onset of free thrombin formation and the velocity of initial thrombin formation are the parameters of the analysis.

**Fig. 2:** Prior art: For the  assessment of the "endogenous thrombin potential" the formation and inhibition of thrombin is calculated. A high amount of a slow thrombin substrate, long measuring times and complex calculation algorithms are required. Several sources of artifacts are caused by the use of a slow substrate and the long detection period.

**Fig 3:** Rise of the optical density by the conversion of a slow chromogenic thrombin substrate according to the prior art. Different substrate concentrations have been used (concentrations shown in the diagram).

**Fig 4:** Prior art: First derivative of the reaction curves shown in Fig. 3.

**Fig. 5:** : Rise of the optical density by the conversion of a fast chromogenic thrombin substrate according to the inventive assay. Different substrate concentrations have been used (concentrations shown in the diagram). A much stronger signal is generated by the inventive assay in comparison to the prior art (see Fig. 3).

**Fig 6:** First derivative of the reaction curve shown in Fig. 5. Using the same substrate concentration a more than 100 times stronger signal is generated when compared to the prior art(see Fig. 4). The curves show much less noise when compared to the prior art.

**Fig. 7:** Effect of the activation of the inventive assay by different concentrations of recombinant tissue factor (concentration shown in the figure in ng/ml).

**Fig 8:** First derivative of the reaction curve shown in Fig. 7.

**Fig. 9:** Prior art: Optical artifacts due to fibrin gelation phenomena.

**Fig. 10:** Prior art: First derivative of the reaction curve shown in Fig. 9.

**Fig. 11:** Correlation of the onset of clotting (detected by the fibrin formation) and the onset of thrombin formation detected by the inventive assay. The onset of free thrombin formation in the inventive assay is detected later than the onset of fibrin formation, however there is a very good correlation of the two methods. The assessment of the plasmatic coagulation factors is thus not disturbed by the inventive assay.

**Fig. 12:** Effect of decreasing activities of the coagulation factor FVIII (activities shown in the figure) on the inventive assay. The calculation of the onset time and the initial thrombin formation is shown in the figure.

**Fig. 13:** Correlation of the factor VIII activity and the initial thrombin formation (assessed according to the inventive assay) in hemophilia A patients and healthy volunteers. The initial thrombin formation was transformed into "% of normal" using a calibration using a normal plasma pool (which was set as 100%).

**Fig. 14:** Effect of the addition of FVIII to hemophilia A patients on the initial thrombin formation (assessed using the inventive method). The initial thrombin formation was transformed into "% of normal" using a calibration using a normal plasma pool (which was set as 100%).

**Fig. 15:** Correlation of the initial thrombin formation (assessed according to the inventive method) and the prothrombin time. Rectangles: assessed according to example 2a, circles: assessed according to example 2b, rhombs: assessed according to example 2c.

**Fig. 16:** Correlation of the initial thrombin formation (assessed according to the inventive method, expressed) to the prothrombin time. Rectangles: assessed according to example 2a, circles: assessed according to example 2b, rhombs: assessed according to example 2c. The initial thrombin formation was transformed into "% of normal" using a calibration using a normal plasma pool (which was set as 100%).

**European Patent Office**

## EUROPEAN SEARCH REPORT

Application Number

EP 02 01 1945

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | RIJKERS D T S ET AL: "DESIGN AND SYNTHESIS OF THROMBIN SUBSTRATES WITH MODIFIED KINETIC PARAMETERS" THROMBOSIS RESEARCH, TARRYTOWN, NY, US, vol. 79, no. 5/6, 15 September 1995 (1995-09-15), pages 491-499, XP002000556 ISSN: 0049-3848 * page 496, paragraph 1 - page 498, last paragraph; figure 1; table 2 * | 1-3, 5-23, 25-29, 31-36 | C12Q1/37 |
| Y | | 4,24,30 | |
| X | LOTTENBERG R ET AL: "THE ACTION OF THROMBIN ON PEPTIDE P-NITROANILIDE SUBSTRATES: HYDROLYSIS OF TOS-GLY-PRO-ARG-PNA AND D-PHE-PIP-ARG-PNA BY HUMAN ALPHA AND GAMMA AND BOVINE ALPHA AND BETA-THROMBINS" THROMBOSIS RESEARCH, TARRYTOWN, NY, US, vol. 28, no. 1, 1 October 1982 (1982-10-01), pages 313-332, XP000196089 ISSN: 0049-3848 * abstract; tables 1-4 * | 1-36 | |
| X | BAUGHMAN D J ET AL: "THROMBIN ACTIVATION RATE CONSTANT 1 STAGE CHROMOGENIC ASSAY FOR THE EXTRINSIC SYSTEM" THROMBOSIS RESEARCH, vol. 26, no. 1, 1982, pages 1-12, XP009000059 ISSN: 0049-3848 * page 6, paragraphs 2,3; figures 1,2 * | 1-36 | |

TECHNICAL FIELDS SEARCHED (Int.Cl.7)

C12Q

-/--

The present search report has been drawn up for all claims

| Place of search | Date of completion of the search | Examiner |
|---|---|---|
| MUNICH | 16 October 2002 | Stachowiak, O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

European Patent
Office

**EUROPEAN SEARCH REPORT**

Application Number

EP 02 01 1945

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | EP 0 420 332 A (HEMKER HENDRIK COENRAAD; BEGUIN SUZETTE LUCETTE (NL)) 3 April 1991 (1991-04-03) * page 4, line 25 - page 6, line 27; claims 1,38; figure 2; examples 1,2 * | 1-36 | |
| Y | DE PEURIOT M D ET AL: "ELECTROCHEMICAL ACTIVITY DETERMINATION OF TRYPSIN-LIKE ENZYMES 4. COUPLED ELECTROCHEMICAL AND SPECTROPHOTOMETRIC ASSAY OF THROMBIN USING THE SAME D PHENYLALANYL PIPECOLYL ARGININE 4 METHOXY-BETA NAPHTHYLAMIDE DI HYDRO CHLORIDE S-2421 SUBSTRATES" THROMBOSIS RESEARCH, vol. 22, no. 3, 1981, pages 303-308, XP009000121 ISSN: 0049-3848 * abstract * | 24 | |
| Y | DE 199 04 674 A (HAEMOSYS GMBH) 31 August 2000 (2000-08-31) * page 3, line 32 * | 30 | TECHNICAL FIELDS SEARCHED (Int.Cl.7) |
| X | EP 0 229 234 A (BOEHRINGER MANNHEIM GMBH) 22 July 1987 (1987-07-22) * page 2, last paragraph - page 4, paragraph 2; claim 3 * | 1-36 | |
| Y | US 4 070 245 A (SVENDSEN LARS GUNDRO) 24 January 1978 (1978-01-24) * abstract; claim 1 * | 4 | |

-/--

The present search report has been drawn up for all claims

| Place of search | Date of completion of the search | Examiner |
|---|---|---|
| MUNICH | 16 October 2002 | Stachowiak, O |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
 
& : member of the same patent family, corresponding
   document

EPO FORM 1503 03.82 (P04C01)

## EUROPEAN SEARCH REPORT

**European Patent Office**

**Application Number**

EP 02 01 1945

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | HEMKER H C ET AL: "THROMBIN GENERATION IN PLASMA: ITS ASSESSMENT VIA THE ENDOGENOUS THROMBIN POTENTIAL" THROMBOSIS AND HAEMOSTASIS, STUTTGART, DE, vol. 74, no. 1, 1 July 1995 (1995-07-01), pages 134-138, XP000195941 ISSN: 0340-6245 * figure 3; table 1 * | 1-36 | |
| A | KIRCHHOF B ET AL: "THROMBIN GENERATION IN PROTHROMBIN COMPLEX PREPARATIONS ITS EFFECT ON A CHROMOGENIC SUBSTRATE FIBRINOGEN AND PLATELETS" THROMBOSIS RESEARCH, vol. 59, no. 3, 1990, pages 541-552, XP009000058 ISSN: 0049-3848 * figures 1-3; table 3 * | 1-36 | |
| A | US 4 508 644 A (EBERLE REINHARD ET AL) 2 April 1985 (1985-04-02) * abstract; example 1; tables 1-9 * | 1-36 | TECHNICAL FIELDS SEARCHED (Int.Cl.7) |
| A | US 6 207 399 B1 (HEMKER HENDRIK COENRAAD ET AL) 27 March 2001 (2001-03-27) * abstract; claims 1,22 * | 1-36 | |
| A | HEMKER H C ET AL: "CONTINUOUS REGISTRATION OF THROMBIN GENERATION IN PLASMA, ITS USE FOR THE DETERMINATION OF THE THROMBIN POTENTIAL" THROMBOSIS AND HAEMOSTASIS, STUTTGART, DE, vol. 70, no. 4, 1993, pages 617-624, XP000567560 ISSN: 0340-6245 * page 619, column 1, line 1 - page 621, last paragraph; figures 1-9; table 1 * | 1-36 | |

-/--

The present search report has been drawn up for all claims

| Place of search | Date of completion of the search | Examiner |
|---|---|---|
| MUNICH | 16 October 2002 | Stachowiak, O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

**European Patent Office**

**EUROPEAN SEARCH REPORT**

Application Number

EP 02 01 1945

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A,D | US 4 289 498 A (BAUGHMAN D JOSEPH ET AL) 15 September 1981 (1981-09-15) * column 7, line 10 - column 13, line 59; claims 1-20; example 3 * | 1-36 | |
| A | CAPPIELLO MARIO ET AL: "Kinetics of human thrombin inhibition by two novel peptide inhibitors (Hirunorm IV and Hirunorm V)." BIOCHEMICAL PHARMACOLOGY, vol. 52, no. 8, 1996, pages 1141-1146, XP001096097 ISSN: 0006-2952 * page 1142, column 1, last paragraph * | 1-36 | |

TECHNICAL FIELDS SEARCHED (Int.Cl.7)

The present search report has been drawn up for all claims

| Place of search | Date of completion of the search | Examiner |
|---|---|---|
| MUNICH | 16 October 2002 | Stachowiak, O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

34

## ANNEX TO THE EUROPEAN SEARCH REPORT
## ON EUROPEAN PATENT APPLICATION NO.                     EP 02 01 1945

This annex lists the patent family members relating to the patent documents cited in the above-mentioned European search report.
The members are as contained in the European Patent Office EDP file on
The European Patent Office is in no way liable for these particulars which are merely given for the purpose of information.

16-10-2002

| Patent document cited in search report | | Publication date | Patent family member(s) | | Publication date |
|---|---|---|---|---|---|
| EP 0420332 | A | 03-04-1991 | NL | 8902406 A | 16-04-1991 |
| | | | AT | 121792 T | 15-05-1995 |
| | | | DE | 69018910 D1 | 01-06-1995 |
| | | | DE | 69018910 T2 | 24-08-1995 |
| | | | DK | 420332 T3 | 03-07-1995 |
| | | | EP | 0420332 A2 | 03-04-1991 |
| | | | ES | 2072970 T3 | 01-08-1995 |
| | | | JP | 3137261 B2 | 19-02-2001 |
| | | | JP | 3236798 A | 22-10-1991 |
| | | | US | 5192689 A | 09-03-1993 |
| DE 19904674 | A | 31-08-2000 | DE | 19904674 A1 | 31-08-2000 |
| | | | AU | 3416200 A | 25-08-2000 |
| | | | WO | 0046602 A2 | 10-08-2000 |
| | | | EP | 1149173 A2 | 31-10-2001 |
| EP 0229234 | A | 22-07-1987 | DE | 3536903 A1 | 16-04-1987 |
| | | | AU | 569433 B2 | 28-01-1988 |
| | | | AU | 6412686 A | 14-05-1987 |
| | | | EP | 0229234 A1 | 22-07-1987 |
| | | | JP | 62093665 A | 30-04-1987 |
| US 4070245 | A | 24-01-1978 | CH | 622286 A5 | 31-03-1981 |
| | | | AT | 349154 B | 26-03-1979 |
| | | | AT | 453876 A | 15-08-1978 |
| | | | AU | 511716 B2 | 04-09-1980 |
| | | | AU | 1495276 A | 22-12-1977 |
| | | | BE | 843245 A1 | 18-10-1976 |
| | | | CA | 1079167 A1 | 10-06-1980 |
| | | | DE | 2627925 A1 | 30-12-1976 |
| | | | DE | 2661080 C2 | 22-03-1990 |
| | | | DK | 278576 A ,B, | 24-12-1976 |
| | | | FR | 2315695 A1 | 21-01-1977 |
| | | | GB | 1553272 A | 26-09-1979 |
| | | | IL | 49774 A | 30-11-1979 |
| | | | IT | 1070002 B | 25-03-1985 |
| | | | JP | 1077606 C | 25-12-1981 |
| | | | JP | 52003494 A | 11-01-1977 |
| | | | JP | 56022519 B | 26-05-1981 |
| | | | LU | 75212 A1 | 17-02-1977 |
| | | | NL | 7606768 A ,B, | 27-12-1976 |
| | | | NO | 762163 A ,B, | 27-12-1976 |
| | | | SE | 430059 B | 17-10-1983 |
| | | | SE | 7607173 A | 24-12-1976 |
| | | | SU | 673165 A3 | 05-07-1979 |
| | | | SU | 1099839 A3 | 23-06-1984 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 02 01 1945

This annex lists the patent family members relating to the patent documents cited in the above-mentioned European search report.
The members are as contained in the European Patent Office EDP file on
The European Patent Office is in no way liable for these particulars which are merely given for the purpose of information.

16-10-2002

| Patent document cited in search report | | Publication date | Patent family member(s) | | Publication date |
|---|---|---|---|---|---|
| US 4508644 | A | 02-04-1985 | DE | 3244030 A1 | 30-05-1984 |
| | | | AT | 51627 T | 15-04-1990 |
| | | | AU | 563001 B2 | 25-06-1987 |
| | | | AU | 2171383 A | 31-05-1984 |
| | | | CA | 1268299 A1 | 24-04-1990 |
| | | | DE | 3381409 D1 | 10-05-1990 |
| | | | DK | 541583 A | 28-05-1984 |
| | | | EP | 0110306 A2 | 13-06-1984 |
| | | | ES | 527539 D0 | 01-09-1984 |
| | | | ES | 8407014 A1 | 16-11-1984 |
| | | | IE | 56344 B1 | 03-07-1991 |
| | | | JP | 1892243 C | 26-12-1994 |
| | | | JP | 6016719 B | 09-03-1994 |
| | | | JP | 59106446 A | 20-06-1984 |
| | | | NZ | 206394 A | 30-06-1988 |
| | | | US | 4665016 A | 12-05-1987 |
| US 6207399 | B1 | 27-03-2001 | AU | 4634896 A | 31-07-1996 |
| | | | DE | 69615339 D1 | 25-10-2001 |
| | | | DE | 69615339 T2 | 04-07-2002 |
| | | | EP | 0802986 A1 | 29-10-1997 |
| | | | ES | 2162025 T3 | 16-12-2001 |
| | | | WO | 9621740 A1 | 18-07-1996 |
| US 4289498 | A | 15-09-1981 | CA | 1136029 A1 | 23-11-1982 |
| | | | DE | 3068833 D1 | 13-09-1984 |
| | | | EP | 0014039 A1 | 06-08-1980 |
| | | | JP | 55124071 A | 24-09-1980 |

EPO FORM P0459

For more details about this annex : see Official Journal of the European Patent Office, No. 12/82